# EUROPÄISCHE PATENTANMELDUNG

(11) **EP 1 728 501 A1**
(43) Veröffentlichungstag der Anmeldung: **06.12.2006**
(21) Anmeldenummer: 06114380.6
(22) Anmeldetag: 23.05.2006
(51) Int. Cl.: A61K 8/64, A61K 8/65, A61K 8/49, A61Q 17/04

(54) **Polypeptidgebundene UV-Lichtschutzfilter in kosmetischen Zubereitungen**

(30) Priorität: 30.05.2005 DE 102005024967
(71) Anmelder: Beiersdorf AG, 20253 Hamburg (DE)
(72) Erfinder: Mundt, Claudia, 28207 Bremen (DE); Steinforth, Melanie, 22303 Hamburg (DE); Hoop, Kerstin, 25421 Pinneberg (DE)

(57) **Zusammenfassung**

Die vorliegende Erfindung betrifft kosmetische Zubereitungen enthaltend mindestens einen UV-A-Lichtschutzfilter, der an ein Polypeptid gebunden ist, sowie mindestens einen UV-B-Lichtschutzfilter und/oder einen Breitbandfilter.

## Beschreibung

Die vorliegende Erfindung betrifft kosmetische Zubereitungen enthaltend mindestens einen UV-A-Lichtschutzfilter, der an ein Polypeptid gebunden ist, sowie mindestens einen UV-B-Lichtschutzfilter und/oder einen Breitbandfilter.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

An kosmetische Zubereitungen, welche die Haut vor den schädlichen Auswirkungen des Sonnenlichtes schützen sollen, werden eine Vielzahl von Anforderungen gestellt, denen die Zubereitungen des Standes der Technik nur unzureichend gleichmäßig gerecht werden. So sollen die Zubereitungen gesundheitlich unbedenklich sein und die Haut über einen möglichst langen Zeitraum wirkungsvoll vor UV-Strahlung schützen. Die UV-Strahlung sollte idealer Weise über den gesamten UV-Bereich in gleichmäßiger Intensität absorbiert werden. Ferner sollen sich die UV-Filter (-kombinationen) stabil in die Zubereitungen einarbeiten lassen und eine hohe Lagerstabilität aufweisen, was insbesondere bei emulsionsförmigen Zubereitungen die Auswahl der UV-Filterkombinationen beschränkt.

Es war daher die Aufgabe der vorliegenden Erfindung, eine neue kosmetische Zubereitung zu entwickeln, die allen diesen Anforderungen möglichst gerecht wird. Insbesondere sollte die zu entwickelnde Zubereitung mit einer geringen Menge an UV-Lichtschutzfiltern eine hohe und über den UV-A und UV-B-Bereich gleichmäßige UV-Filterleistung erzielen.

Überraschend gelöst wird die Aufgabe durch eine kosmetische Zubereitung enthaltend
a) mindestens einen UV-A-Lichtschutzfilter, der an ein Polypeptid gebunden ist,
b) mindestens einen UV-B-Lichtschutzfilter und/oder einen Breitbandfilter.

Ferner wird die Aufgabe gelöst durch die Verwendung eines UV-A-Lichtschutzfilters, der an ein Polypeptid gebunden ist, zur Erhöhung der UV-B-Absorption von kosmetischen Zubereitungen mit einem Gehalt an UV-B-Lichtschutzfiltern und/oder Breitbandfiltern.

Durch die Verwendung eines UV-A-Lichtschutzfilters, der an ein Polypeptid gebunden ist, wird erfindungsgemäß die UV-A-Balance (gemessen nach der Methode von Wendel et.al., "A new in vitro test method to assess the UVA protection performance of sun care products"; SÖFW-Journal, 127) deutlich erhöht (UV-A-Balance größer/gleich 25).

Auch lässt sich durch die erfindungsgemäße Verwendung eines UV-A-Lichtschutzfilters, der an ein Polypeptid gebunden ist, der SPF (sun protecting factor) signifikant erhöhen.

Der SPF wird dabei erfindungsgemäß mit der in-vivo-Methode gemessen, die durch die "International Sun Protection Factor (SPF) Test Method. CTFA South Africa, COLIPA and JCIA, February 2003" festgelegt ist.

Als "Breitbandfilter" werden erfindungsgemäß UV-Lichtschutzfiltersubstanzen bezeichnet, die sowohl UV-A- als auch UV-B-Strahlung absorbieren.

Der Begriff "erfindungsgemäß Zubereitungen" bezieht sich im Rahmen dieser Offenbarung sowohl die erfindungsgemäßen Zubereitungen als auch die erfindungsgemäßen Verwendungen.

Zwar sind dem Fachmann UV-Lichtschutzfilter, die an ein Polypeptid gebunden sind, an sich bekannt. So beschreiben die WO 2004/075871 und die WO 03/004061 kosmetische Zubereitungen mit einem Gehalt an polypeptid-gebundenen UV-Lichtschutzfiltern. Diese Schriften offenbaren jedoch nicht die Kombination dieser Filter mit UV-B- bzw. Breitbandfiltern, noch wurde dem Fachmann durch diese Schriften nahe gelegt, dass sich durch den Einsatz der Polypeptid-gebundenen UV-A-Lichtschutzfilter, die selbst nicht im UV-B-Bereich absorbieren, die UV-Filterleistung im UV-B-Bereich erhöhen lässt (siehe Vergleichsversuch).

Erfindungsgemäß vorteilhafte Ausführungsformen der vorliegenden Erfindung sind dadurch gekennzeichnet, dass der UV-A-Filter, welcher an das Polypeptid gebunden ist, gewählt wird aus der Gruppe der Verbindungen Rutin, der Benzotriazole und/oder der Merocyanine.
Erfindungsgemäß besonders bevorzugt ist dabei der Einsatz von Merocyanin.

Erfindungsgemäß vorteilhafte Zubereitungen oder Verwendungen sind dadurch gekennzeichnet, dass Gelantine als Polypeptid eingesetzt wird.

Auch ist es vorteilhaft im Sinne der vorliegenden Erfindung, wenn zwischen 0,01 mmol und 100 mmol UV-A-Lichtschutzfilter pro Gramm Polypeptid an das Polypeptid gebunden ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.
Die UV-B- und/oder Breitband-Filter können öllöslich oder wasserlöslich sein. Vorteilhafte öllösliche UV-B- und/oder Breitband-Filtersubstanzen sind z. B.:
■ 2,4,6-Trianilino-(p-carbo-2'-ethyl-1'-hexyloxy)-1,3,5-triazin;
■ 4,4',4"-(1,3,5-Triazin-2,4,6-triyltriimino)-tris-benzoësäure-tris(2-ethylhexylester)
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Vorteilhafte Breitbandfilter oder UV-B-Filtersubstanzen sind beispielsweise auch Bis-Resorcinyltriazinderivate mit der folgenden Struktur: 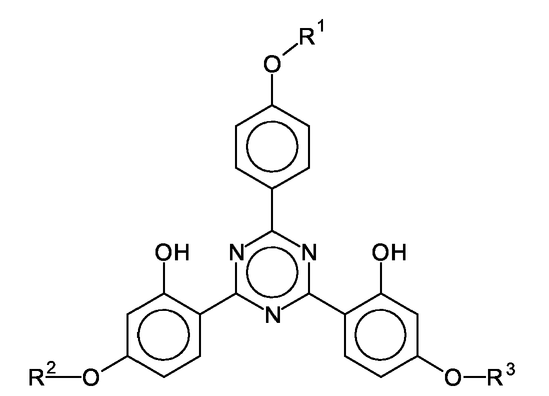 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Die Liste der genannten Filter soll selbstverständlich nicht limitierend sein.

Es ist erfindungsgemäß besonders bevorzugt, wenn als UV-B-Lichtschutzfilter bzw. Breitbandfilter eine oder mehrere Verbindungen gewählt aus der Gruppe der Triazine eingesetzt werden.

Erfindungsgemäß vorteilhafte Zubereitungen oder Verwendungen sind dadurch gekennzeichnet, dass das Gewichtsverhältnis der Gesamtmenge an UV-A-Lichtschutzfilter, der an ein Polypeptid gebunden ist, zur Gesamtmenge an UV-B-Lichtschutzfilter und Breitbandfilter von 0,1:10 bis 10:1 und bevorzugt von 1:5 bis 5:1 beträgt.

Es ist erfindungsgemäß vorteilhaft, wenn die kosmetische Zubereitung UV-A-Lichtschutzfilter, welcher an ein Polypeptid gebunden ist, in einer Menge von 0,1 bis 20 Gewichts-% und bevorzugt, wenn die kosmetische Zubereitung UV-A-Lichtschutzfilter, welcher an ein Polypeptid gebunden ist, in einer Menge von 1 bis 15 Gewichts-%, jeweils bezogen auf das Gesamtgewicht der Zubereitung, enthält.

Es ist erfindungsgemäß vorteilhaft, wenn die kosmetische Zubereitung UV-B-Lichtschutzfilter und/oder Breitbandfilter in einer Gesamtmenge von 0,1 bis 30 Gewichts-%, bezogen auf das Gesamtgewicht der Zubereitung, enthält. Dabei ist ein Konzentrationsbereich von 0,5 bis 20, bezogen auf das Gesamtgewicht der Zubereitung, erfindungsgemäß bevorzugt.

Die erfindungsgemäßen Zubereitungen können in Form einer wässrigen oder wässrigalkoholischen Lösung, einer Emulsion vom Typ Wasser-in-ÖI (W/O) oder vom Typ ÖI-in-Wasser (O/W), oder einer multiple Emulsionen, beispielsweise vom Typ Wasser-in-ÖI-in-Wasser (W/O/W) oder ÖI-in-Wasser-in-ÖI (O/W/O), einer Hydrodispersion oder Lipodispersion, eines Gels, einer Pickering-Emulsion, eines festen Stiftes oder auch eines Aerosols vorliegen.

In einer erfindungsgemäß besonders bevorzugten Ausführungsform liegt die kosmetische Zubereitung in Form einer O/W-Emulsion vor.

Die erfindungsgemäße Zubereitung kann neben der erfindungsgemäßen UV-Filterkombination zusätzlich noch weitere UV-Lichtschutzfiltersubstanzen enthalten.

Vorteilhafte bei Raumtemperatur flüssige UV-Filtersubstanzen im Sinne der vorliegenden Erfindung sind Homomenthylsalicylat (INCl: Homosalate), 2-Ethylhexyl-2-hydroxybenzoat (2-Ethylhexylsalicylat, Octylsalicylat, INCl: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCl: Octyl Methoxycinnamate) und 4-Methoxyzimtsäureisopentylester (Isopentyl-4-methoxycinnamat, INCl: Isoamyl p-Methoxycinnamate), 3-(4-(2,2-bis Ethoxycarbonylvinyl)-phenoxy)propenyl)-methoxysiloxan/ Dimethylsiloxan ― Copolymer (INCl: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Die Zubereitung kann auch Lichtschutzfilterpigmente enthalten.

Es ist dabei erfindungsgemäß von Vorteil, wenn die anorganischen UV-Lichtschutzfilterpigmente gewählt werden aus der Gruppe der folgenden Pigmente:

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 200 nm aus, wobei Partikelgrößen von 10 nm bis 100 nm erfindungsgemäß bevorzugt sind.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eusolex TS | Alumina, Stearinsäure - | | Merck KgaA |
| Eusolex T-AVO | Silica | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |
| Titandioxid T-817 (Eisen/TitanMischo xid) | Eisenoxid | | Degussa |

Im Sinne der vorliegenden Erfindung sind besonders bevorzugte Titandioxide das MT-100 Z und MT-100 TV von Tayca Corporation, Eusolex T-2000 und Eusolex T-AVO von Merck und das Titandioxid T 805 von Degussa und das Eisen/Titandmischoxid Titandioxid T817 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Ferner kann die erfindungsgemäße Zubereitung organische Lichtschutzfilterpigmente enthalten, beispielsweise 2,4,6-Tris-(biphenyl)-1,3,5-triazin und 2,4,6-Tris-(terphenyl)-1,3,5-triazin. Dabei ist es erfindungsgemäß bevorzugt, 2,4,6-Tribiphenyl-4-yl-1,3,5-triazin als organisches Lichtschutzfilterpigment einzusetzen.

Ferner kann die erfindungsgemäße Zubereitung weitere UV-A-Lichtschutzfilter enthalten, beispielsweise:
- 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird,
- 3, 3'-(1,4-Phenylendimethin)-bis (*l*,7-dimethyl-2-oxo-bicyclo[2.2.1]heptan-1methansulfonsäure und ihre Salze (erhältlich bei Chimex unter dem Handelsnamen Mexoryl SX),
- 2-(4'-Diethylamino-2'-hydoxybenzoyl)-benzoesäurehexylester (auch: Aminobenzophenon), welches unter dem Namen Uvinul A Plus bei der Fa. BASF erhältlich ist,
- 4-(tert.-Butyl)-4'-methoxydibenzoylmethan (INCl: Butylmethoxydibenzoylmethan, Handelsname: Parsol 1789),
- 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A).

Die Lipidphase (Ölphase) kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl, Macadamia-, Avocado- oder Jojobaöl, Dialkylether wie z.B. Di-n-octylether sowie Dialkylcarbonate wie beispielsweise Di-n-octylcarbonat
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe lsopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Phenethyl Benzoat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäuretriglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alky-Ibenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die erfindungsgemäße Zubereitung bzw. erfindungsgemäße Verwendungen sind erfindungsgemäß vorteilhaft dadurch gekennzeichnet, dass sie 2-Phenylethylbenzoat in einer Konzentration von 0,5 bis 20 Gewichts-% und erfindungsgemäß bevorzugt von 2 bis 10 Gewichts-%, jeweils bezogen auf das Gesamtgewicht der Zubereitung, enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Propandiol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind Permulen TR 1, TR 2, Carbopol 1328, Aristoflex AVC.

Die erfindungsgemäßen kosmetischen Zubereitungen können wie üblich zusammengesetzt sein. Besonders vorteilhaft im Sinne der vorliegenden Erfindung sind Zubereitungen zur Pflege der Haut: sie können dem kosmetischen Lichtschutz, ferner zur Reinigung oder Pflege der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Zur Anwendung werden die erfindungsgemäßen kosmetischen Zubereitungen in der für Kosmetika üblichen Weise auf die Haut und/oder die Haare in ausreichender Menge aufgebracht.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, Niacinamid, Panthenol, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCl-Bezeichnung Octadecendioic acid) und/oder Licochalcon A.

Licochalcon kann vorteilhaft auch als Bestandteil von pflanzlichen Extrakten, insbesondere von wäßrigen *Radix Glycyrrhizae inflatae,* eingesetzt werden.

Es ist erfindungsgemäß vorteilhaft, wenn die kosmetischen Zubereitungen 0,001 bis 10 Gew.%, insbesondere 0,05 bis 5 Gew.-%, ganz besonders 0,01 bis 2 Gew.-% an einem Extrakt aus *Radix Glycyrrhizae inflatae* enthalten, jeweils bezogen auf das Gesamtgewicht der Zubereitung.

Ganz besonders vorteilhaft ist es, von einem Extrakt auszugehen, der unter der Bezeichnung Polyol Soluble Licorice Extract PU (INCI-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcone A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Methylpropandiol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz kosmetischer Hautveränderungen, wie sie z. B. bei der Hautalterung auftreten, verwendet werden.

Die erfindungsgemäße UV-Lichtschutzfilterkombination aus
a) mindestens einen UV-A-Lichtschutzfilter, der an ein Polypeptid gebunden ist,
b) mindestens einen UV-B-Lichtschutzfilter und/oder einen Breitbandfilter
und die erfindungsgemäßen kosmetischen Zubereitungen, welche diese Kombination enthalten, können erfindungsgemäß vorteilhaft für eine Reihe von weiteren Anwendungen eingesetzt werden:
- Erfindungsgemäß ist die Verwendung der erfindungsgemäßen UV-Lichtschutzfilterkombination und der erfindungsgemäßen kosmetischen Zubereitungen zur Prophylaxe und Behandlung von Hautalterungserscheinungen (insbesondere Falten, Altersflecken, Teleangiektasien), lichtbedingten Hautschäden, Pigmentstörungen und polymorpher Lichtdermatose.
- Erfindungsgemäß ist die Verwendung der erfindungsgemäßen UV-Lichtschutzfilterkombination zur Herstellung eines Kosmetikums zur Prophylaxe und Behandlung von Hautalterungserscheinungen (insbesondere Falten, Altersflecken, Teleangiektasien), lichtbedingten Hautschäden, Pigmentstörungen und polymorpher Lichtdermatose.

Überraschend an diesen Verwendungen war insbesondere der Umstand, dass die erfindungsgemäßen Zubereitungen im Vergleich zu Zubereitungen des Standes der Technik deutlich wirkungsvoller sind. So wird mit den erfindungsgemäßen Zubereitungen bei gleichem SPF und/oder gleicher UV-A-Balance ein deutlich höherer Schutz vor Hautalterungserscheinungen (insbesondere Falten, Altersflecken, Teleangiektasien), lichtbedingten Hautschäden, Pigmentstörungen und polymorpher Lichtdermatose erreicht als dies mit Zubereitungen des Standes der Technik möglich ist.

Es ist dabei zu berücksichtigen, dass "Prophylaxe" und "Behandlung" im Rahmen dieser Offenbarung ausschließlich die kosmetische Prophylaxe und Behandlung und keinesfalls eine therapeutische Prophylaxe und Behandlung im Sinne des Patentrechtes bedeuten.

Erfindungsgemäß bevorzugt sind diese Verwendungen dann, wenn das Kosmetikum bzw. die kosmetische Zubereitung topisch auf der Haut angewendet wird.

Darüber hinaus kann mit den erfindungsgemäßen Zubereitungen und UV-Lichtschutzfilterkombinationen bei der Anwendung auf dem Haar eine deutliche Reduzierung des Ausbleichens des Haares sowie, bei gefärbten Haaren, ein deutlich besserer Schutz der Haarfärbung erreicht werden, wenn das Haar dem Sonnenlicht ausgesetzt wird.
Ebenso werden permanente Farbmuster auf der Haut, so genannte "Tatoos" vor Verfärbungen wirkungsvoll geschützt.

### Vergleichsversuch: Erhöhung des SPF durch gelatinegekoppelte UV-Filter

Der folgende Vergleichsversuch belegt den erfinderischen Effekt: :
a) Absorptionsspektrum von an Gelantine gebundenem Merocyanin 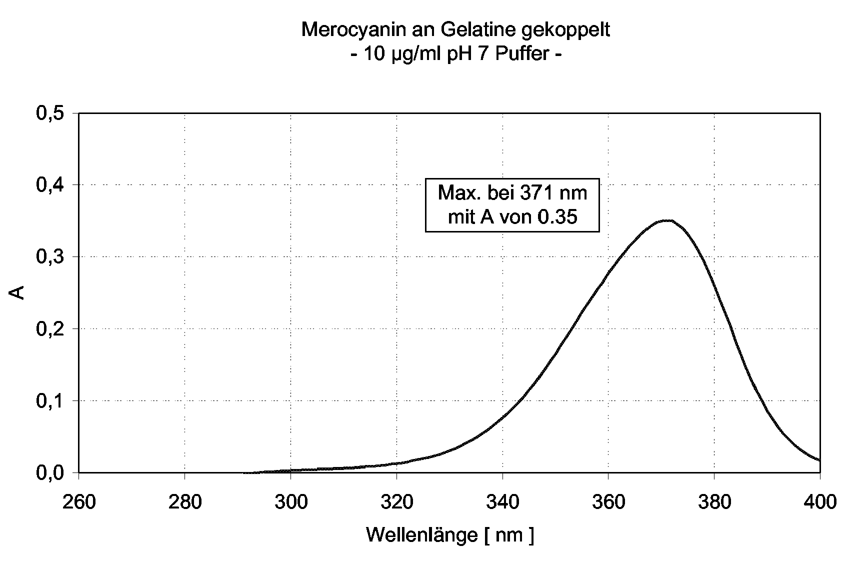 Das Spektrum zeigt, dass der an Gelantine gebundene UV-Filter ausschließlich im UV-A-Bereich absorbiert. Das Absorbtionsmaximum liegt bei 370 nm.
b) Absorptionsspektrum des UV-B-Filters Octyl Triazone. 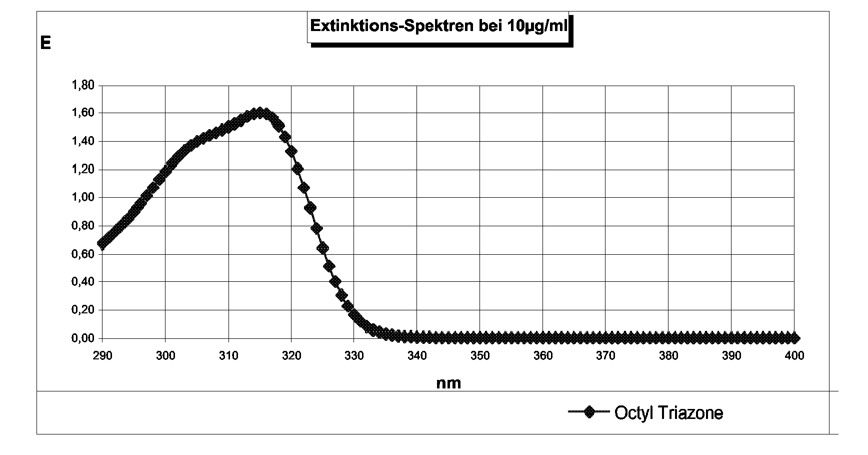 Das Spektrum zeigt, dass der UV-B-Filter ausschließlich im UV-B-Bereich absorbiert.

Es wurden nun die beiden folgenden kosmetischen Rezepturen mit dem UV-B-Filter Octyl Triazone hergestellt. Rezeptur B unterscheidet sich von Rezeptur A allein darin, dass sie zusätzlich an Gelantine gebundenenes Merocyanin enthält und damit die erfindungsgemäße Zubereitung darstellt.

| | **A** | **B** |
|---|---|---|
| l**NCl-Name(n)** | **m [%]** | **m [%]** |
| Merocyanin an Gelatine gekoppelt | | 6,00 |
| Alcohol Denat. | 4,00 | 4,00 |
| Bis-Ethylhexyloxyphenol Methoxyphenyl Triazine | 2,00 | 2,00 |
| Butyl Methoxydibenzoylmethane | 2,00 | 2,00 |
| Butylene Glycol Dicaprylate/Dicaprate | 7,50 | 7,50 |
| C12-15 Alkyl Benzoate | 7,50 | 7,50 |
| Ceteareth-20 | 1,00 | 1,00 |
| Cetearyl Alcohol + PEG-40 Castor Oil + Sodium Cetearyl Sulfate | 2,00 | 2,00 |
| Cetyl Alcohol | 1,50 | 1,50 |
| Dicaprylyl Carbonate | 3,00 | 3,00 |
| Diethylhexyl Butamido Triazone | 1,50 | 1,50 |
| Dimethicone | 3,00 | 3,00 |
| Ethylhexyl Triazone | 1,50 | 1,50 |
| Ethylparaben | 0,10 | 0,10 |
| Parfüm | 0,40 | 0,40 |
| Glycerin | 6,00 | 6,00 |
| Glyceryl Stearate SE | 0,70 | 0,70 |
| Hydrogenated Coco-Glycerides | 0,50 | 0,50 |
| Methylparaben | 0,30 | 0,30 |
| Phenoxyethanol | 0,60 | 0,60 |
| Propylparaben | 0,10 | 0,10 |
| Trinatrium EDTA | 1,00 | 1,00 |
| Natriumhydroxid 45 % | 0,06 | 0,06 |
| Xanthangummi | 0,40 | 0,40 |
| Wasser | ad 100 | ad 100 |
| | | |
| **In-Vivo SPF** | **7** | **16** |
| **UVA- Balance** | **59** | **75** |

Alle Mengenangaben in Gew.-%.

Für beide Zubereitungen wurde der SPF und die UV-A-Balance bestimmt. Der SPF wurde dabei mit der in-vivo-Methode gemessen, die durch die "International Sun Protection Factor (SPF) Test Method. CTFA South Africa, COLIPA and JCIA, February 2003" festgelegt ist.

Die Höhe des SPF hängt bei dieser Messmethode naturgemäß von der UV-B-Filterleistung der Zubereitung ab.

Obwohl der in der Zubereitung B zusätzliche an Gelantine gebundene UV-Filter ausschließlich im UV-A-Bereich absorbiert (siehe a) Absorptionsspektrum von an Gelantine gebundenem Merocyanin) führt sein Einsatz zur einer Erhöhung des SPF. Dieser Effekt war für den Fachmann unerwartet.

### Beispielrezepturen

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### 1. Beispiele für W/O-Emulsionen

| **Emulsion** | **A** | **B** | **C** | **D** | **E** | **F** |
|---|---|---|---|---|---|---|
| Polyglyceryl-2 Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | | 0,3 | | 1 |
| Glycin | 0,3 | 0,3 | 0,5 | 0,4 | | |
| Alkohol | | 5 | 2 | 5 | 4 | |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid | 5 | 6 | 4 | | | 4 |
| Zinkoxid | 5 | | | | | |
| Bis-Ethylhexyloxyphenol Methoxyphenyltriazin | | 3 | 3 | 2 | | |
| Ethylhexyltriazon | | 4,5 | 3 | | 3 | |
| Diethylhexylbutamidotriazon | | | 1,5 | 4 | | |
| Butyl Methoxydibenzoylmethan | 2 | 3 | 4 | | 1 | 3 |
| Uvinul A Plus® | | | | 4 | 2 | |
| Ethylhexylmethoxycinnamat | | | | | 7 | 5 |
| Merocyanin an Gelatine gekoppelt | 2 | 5 | | 10 | 5 | 8 |
| Benzotriazol an Gelatine gekoppelt | 4 | | 6 | | | |
| Taurin | 0,1 | | | 0,5 | 0,2 | |
| Vitamin E Acetat | 0,2 | 0,2 | | 0,3 | 0,1 | 0,5 |
| Na₂H₂EDTA | 0,1 | 0,1 | 0,2 | 0,2 | 0,2 | 0,5 |
| C8-C16 Alkylpolyglycosid | 1 | | | | | |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

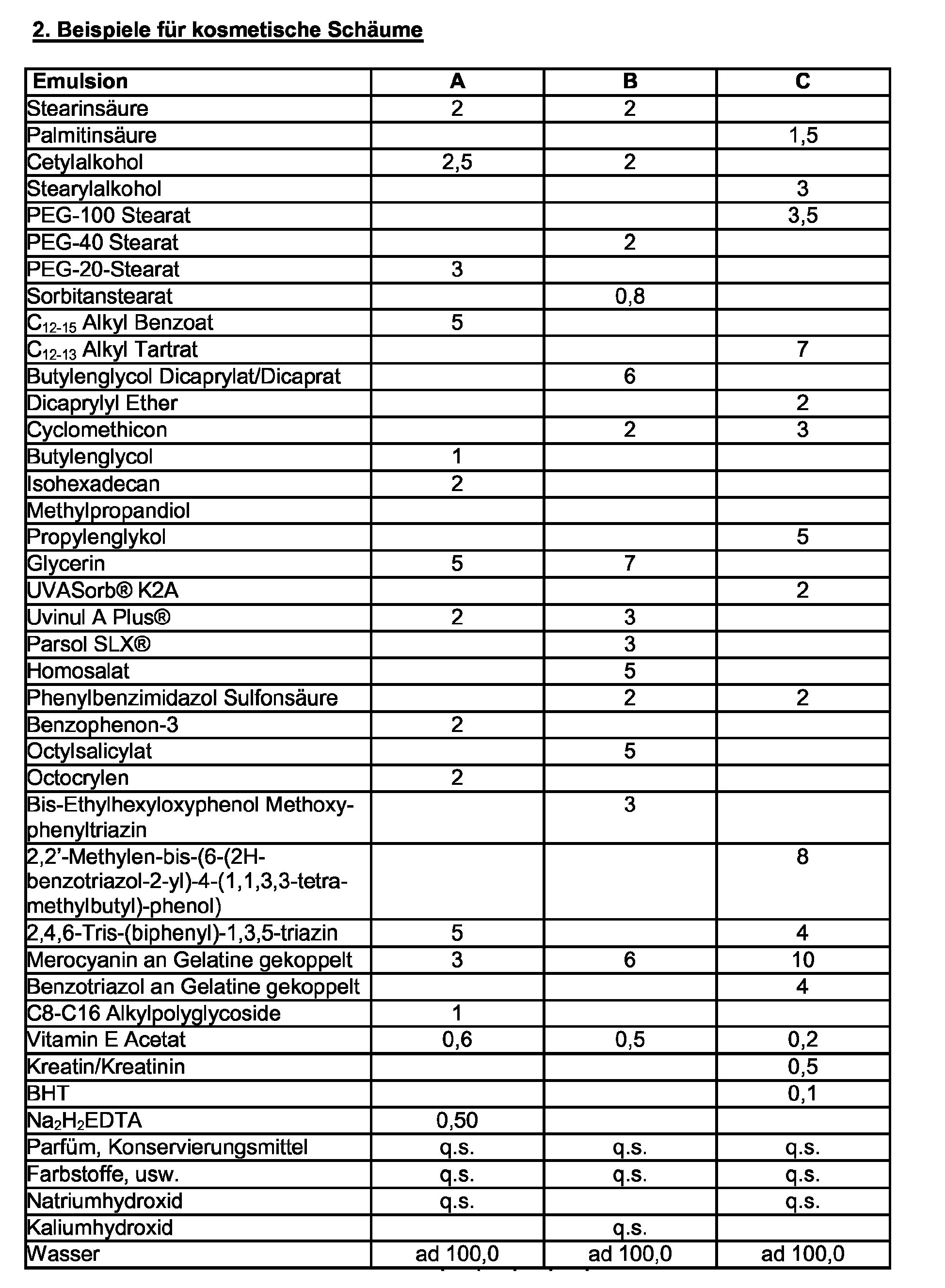

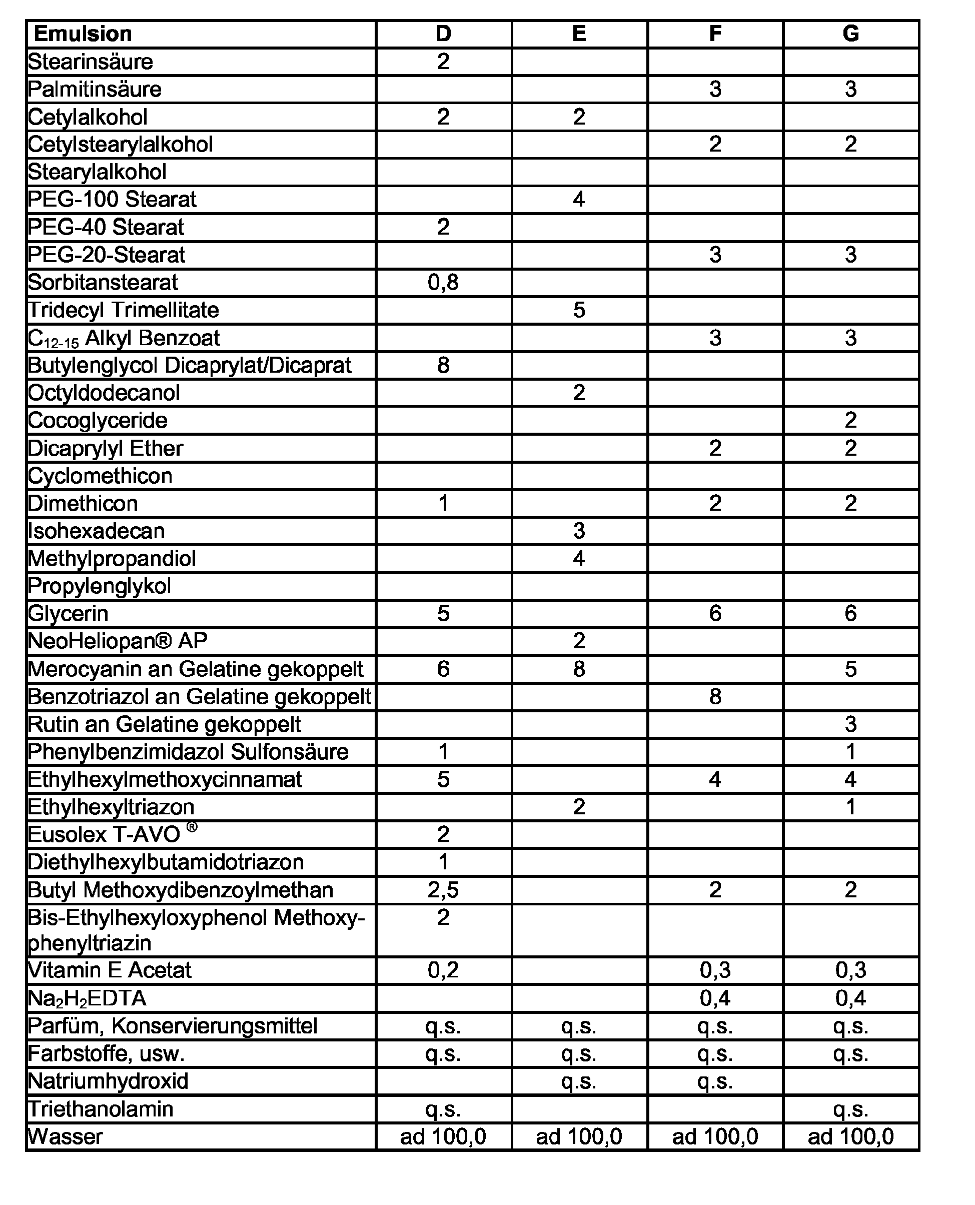

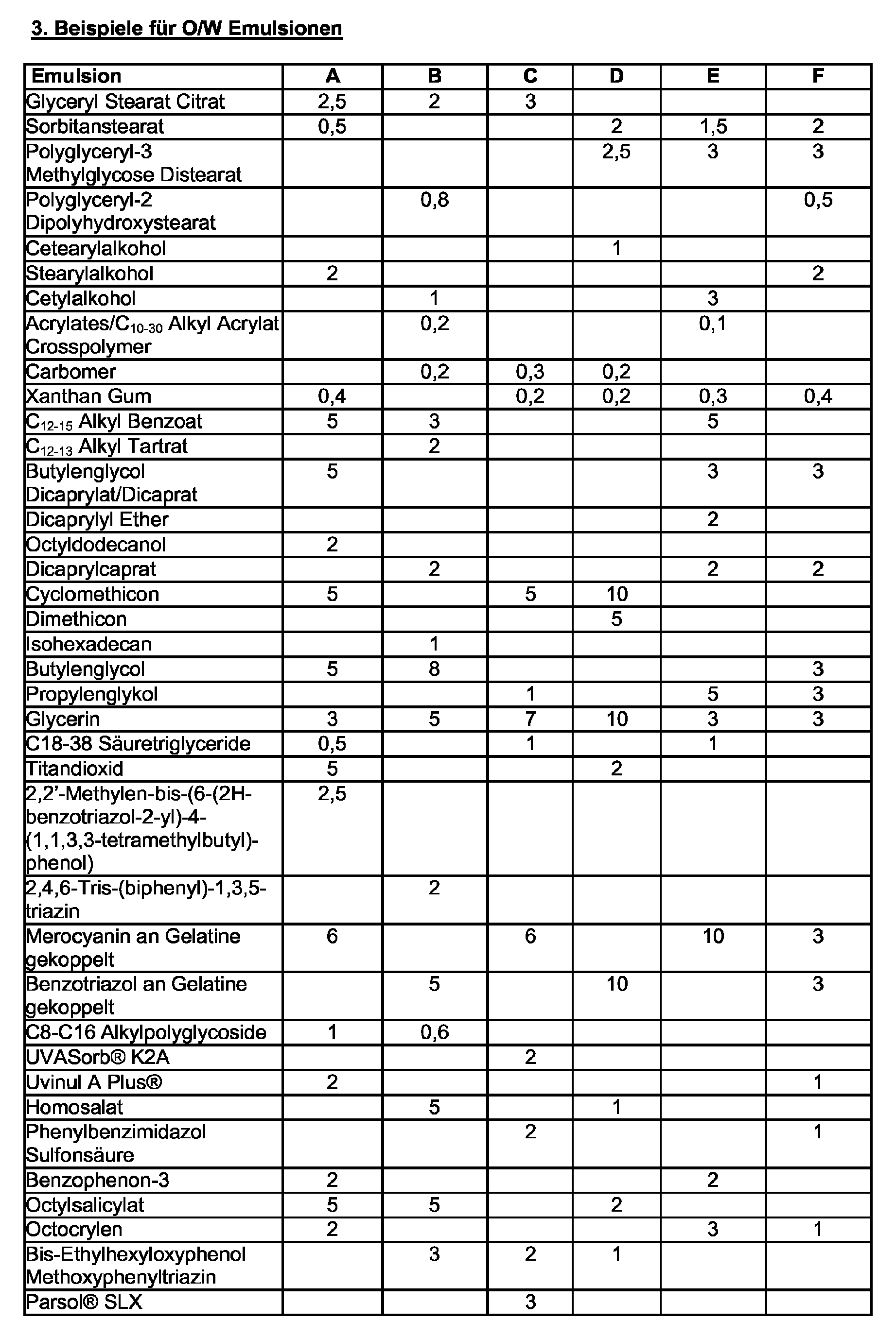

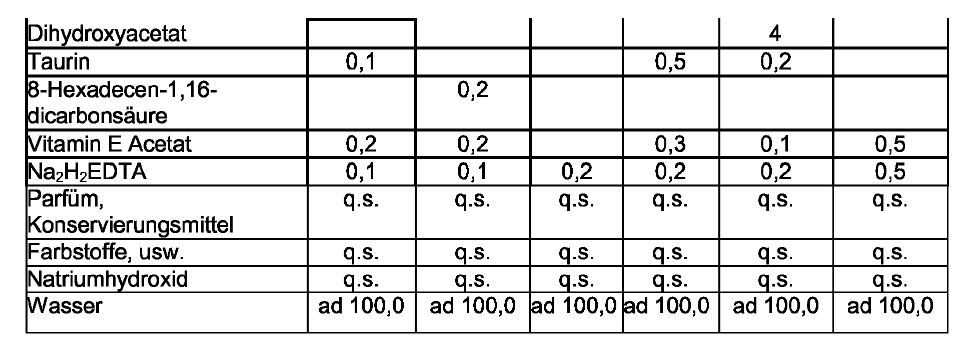

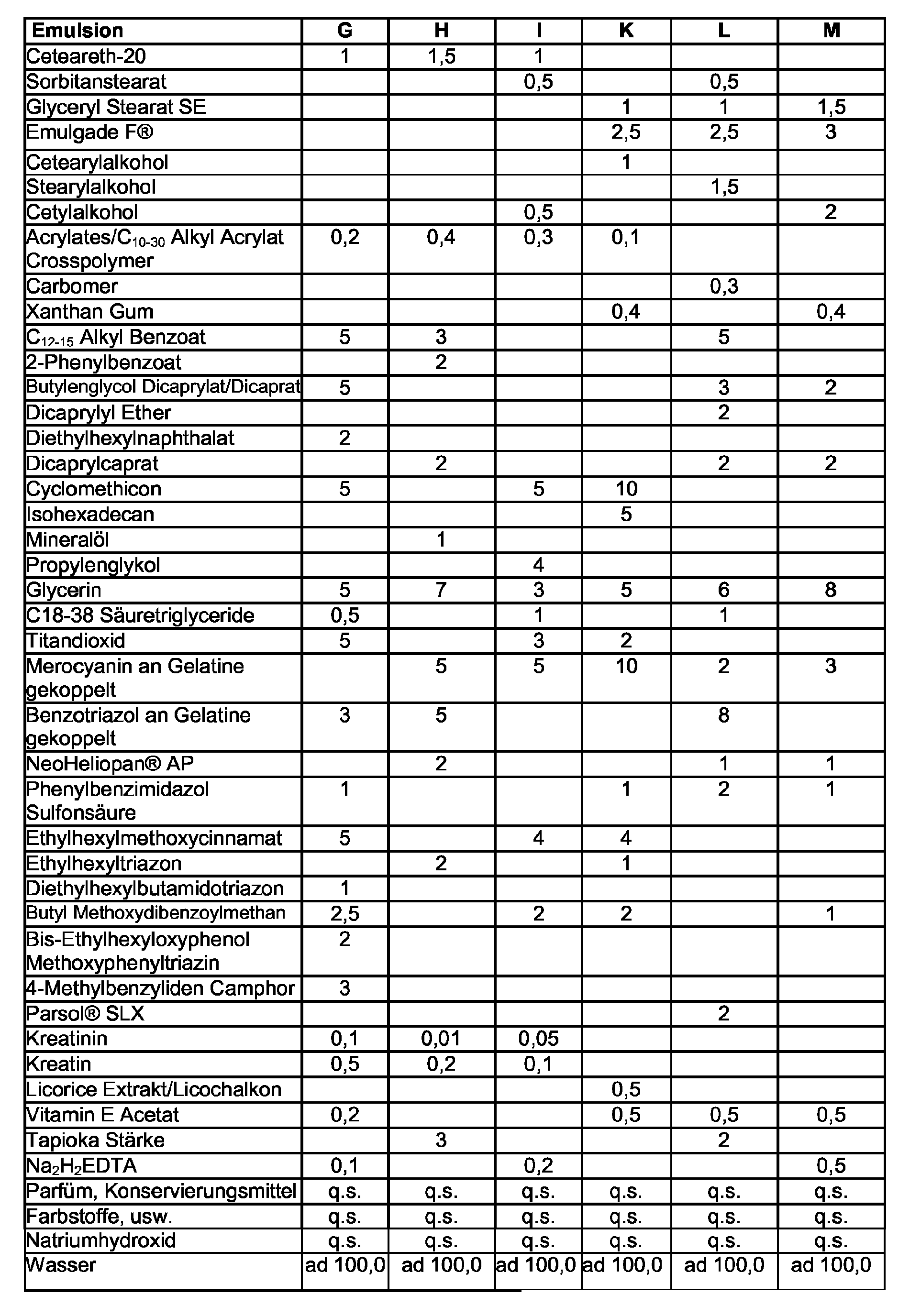

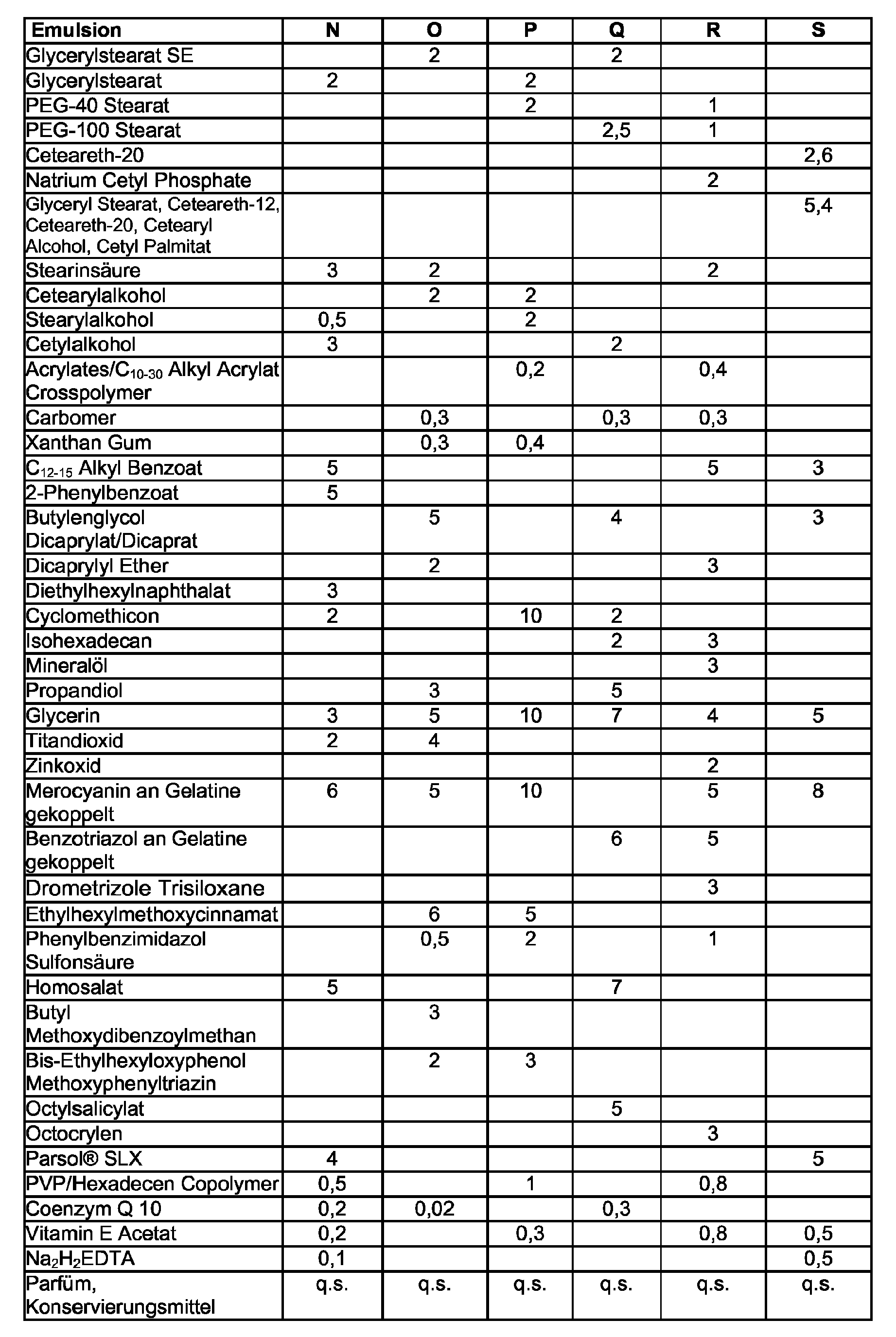

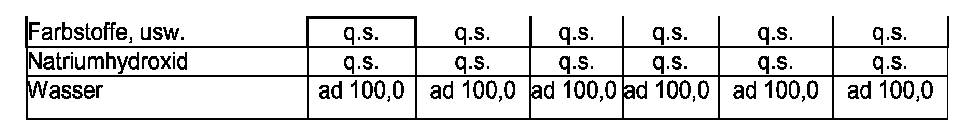

### 4. Beispiel : Hydrodispersion (zur Verwendung als Lotion oder Spray)

| | **A** | **B** | **C** | **D** | **E** | **F** |
|---|---|---|---|---|---|---|
| Glyceryl Stearat Citrat | | 0,40 | | | | |
| Cetyl Alkohol | | | | | 2,00 | |
| Natrium Carbomer | | | | | 0,30 | |
| Acrylates/C10-30 Alkyl Acrylate Crosspolymer | 0,30 | | 0,30 | 0,40 | 0,10 | 0,10 |
| Ceteareth-20 | | | 1,00 | | | |
| Xanthan Gummi | | | | 0,15 | | 0,50 |
| Dimethicon / Vinyl Dimethicon Crosspolymer | | | | 5,00 | | 3,00 |
| UVASorb® K2A | | | | | 3,50 | |
| Uvinul®A Plus | 0,25 | | | 0,50 | 2,00 | 1,50 |
| Butyl Methoxydibenzoylmethan | 1,20 | | 3,50 | | | |
| Bis-Ethylhexyloxyphenol Methoxypheny Triazin | 2,00 | 2,00 | | 0,25 | | |
| Terephthaliden Dicampher Sulfonsäure | | | | | | 0,50 |
| Dinatrium Phenyl Dibenzimidazol Tetrasulfonat | | | | | | 1,00 |
| Phenylbenzimidazol Sulfonsäure | | | 2,00 | | | |
| Ethylhexyl Methoxycinnamat | 5,00 | | 7,00 | | 5,00 | 8,00 |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| Merocyanin an Gelatine gekoppelt | 5,00 | 10,00 | 4,00 | 3,00 | | 5,00 |
| Benzotriazol an Gelatine gekoppelt | | | | 3,00 | 10,00 | 5,00 |
| C₁₂₋₁₅ Alkyl Benzoat | 2,00 | | 2,50 | | | |
| Phenethyl Benzoat | 4,00 | | | 7,50 | | 5,00 |
| C18-36 Triglycerid Fettsäure | | | 1,00 | | | |
| Butylenglycol Dicaprylat/Dicaprat | | | | | 6,00 | |
| Dicaprylyl Carbonat | | 3,00 | | | | |
| Dicaprylylether | | 2,00 | | | | |
| Cyclomethicon | | | | 1,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α - Glycosylrutin | | | | | 0,25 | |
| Trinatrium EDTA | | 1,00 | 1,00 | 0,10 | 0,20 | |
| lodopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |

### 5. Beispiel: wässrige bzw. wässrig/alkoholische Formulierungen

| | **A** | **B** | **C** | **D** | **E** | **F** |
|---|---|---|---|---|---|---|
| Ethanol | 50 | 5 | 2 | 40 | 15 | |
| Hydroxyethylcellulose | 0,5 | | | | | |
| Acrylates/C10-30 Alkyl Acrylate Crosspolymer | | | | 0,3 | 0,6 | |
| Cocoamidopropylbetain | | | 0,3 | | | |
| UVASorb® K2A | | | | | 2 | |
| Uvinul® A Plus | 5 | | | | | |
| Butyl Methoxydibenzoylmethan | 0,5 | | | 3 | | |
| Dinatrium Phenyl Dibenzimidazol Tetrasulfonat | | 2 | 1 | | | |
| Phenylbenzimidazol Sulfonsäure | | 5 | 3 | | 2 | 4 |
| Ethylhexyl Methoxycinnamat | 10 | | | | 3 | |
| Diethylhexyl Butamido Triazon | | | | 3 | | |
| Ethylhexyl Triazon | | | | | 2 | |
| Octocrylen | | | | 5 | | |
| Merocyanin an Gelatine gekoppelt | 5 | 6 | | 3 | | 10 |
| Benzotriazol an Gelatine gekoppelt | | | 5 | 3 | | |
| C₁₂₋₁₅ Alkyl Benzoat | | | | 3 | | |
| C18-36 Triglycerid Fettsäure | | | | 1 | | |
| Butylenglycol Dicaprylat/Dicaprat | 2 | | | | | |
| C12-13 Alkyl Tartrat | | | | | 5 | |
| Cyclomethicon | 4 | | | 2 | | |
| Insekt Repellent® 3535 | | | | 5 | | |
| Dimethicon | | | | | 3 | |
| PVP Hexadecen Copolymer | | 0,5 | | 1 | | 0,5 |
| Ethylhexyloxyglycerin | | 0,5 | | | | |
| Glycerin | 5 | 7 | 3 | 8 | | 5 |
| Butylenglycol | | | 5 | | 5 | |
| Metylpropandiol | | | | 4 | | |
| Vitamin E Acetat | | 0,3 | 0,2 | 0,5 | | |
| Panthenol | 0,5 | | 0,2 | | | 0,3 |
| Kreatinin | | | 0,01 | | 0,02 | |
| Creatin | | | 0,1 | | 0,2 | |
| PEG-40 Hydriertes Ricinusöl | | 0,5 | 0,3 | | | 0,5 |
| Trinatrium EDTA | 0,3 | 0,2 | 0,2 | 0,2 | 0,2 | 0,5 |
| Konservierungsmittel | q.s. | q.s. | q.s. | q.s. | q.s. | q.s. |
| Natriumhydroxid | q.s. | q.s. | q.s. | q.s. | q.s. | q.s. |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Zubereitung enthaltend
a) mindestens einen UV-A-Lichtschutzfilter, der an ein Polypeptid gebunden ist,
b) mindestens einen UV-B-Lichtschutzfilter und/oder einen Breitbandfilter.

2. Verwendung eines UV-A-Lichtschutzfilters, der an ein Polypeptid gebunden ist, zur Erhöhung der UV-B-Absorption von kosmetischen Zubereitungen mit einem Gehalt an UV-B-Lichtschutzfiltern und/oder Breitbandfiltern.

3. Zubereitung oder Verwendung nach einem der Ansprüche 1 oder 2, **dadurch gekennzeichnet, dass** der UV-A-Filter, welcher an das Polypeptid gebunden ist, gewählt wird aus der Gruppe der Verbindungen Rutin, der Benzotriazole und/oder der Merocyanine.

4. Zubereitung oder Verwendung nach einem der vorhergehenden Ansprüche, **dadurch gekennzeichnet, dass** Gelantine als Polypeptid eingesetzt wird.

5. Zubereitung oder Verwendung nach einem der vorhergehenden Ansprüche, **dadurch gekennzeichnet, dass** zwischen 0,01 mmol und 100 mmol UV-A-Lichtschutzfilter pro Gramm Polypeptid an das Polypeptid gebunden ist.

6. Zubereitung oder Verwendung nach einem der vorhergehenden Ansprüche, **dadurch gekennzeichnet, dass** als UV-B-Lichtschutzfilter bzw. Breitbandfilter eine oder mehrere Verbindungen gewählt aus der Gruppe der Triazine eingesetzt werden.

7. Zubereitung oder Verwendung nach einem der vorhergehenden Ansprüche, **dadurch gekennzeichnet, dass** das Gewichtsverhältnis der Gesamtmenge an UV-A-Lichtschutzfilter, der an ein Polypeptid gebunden ist, zur Gesamtmenge an UV-B-Lichtschutzfilter und Breitbandfilter von 0,1 : 10 bis 10 : 1 beträgt.

8. Zubereitung oder Verwendung nach einem der vorhergehenden Ansprüche, **dadurch gekennzeichnet, dass** die kosmetische Zubereitung UV-A-Lichtschutzfilter, welcher an ein Polypeptid gebunden ist, in einer Menge von 0,1 bis 20 Gewichts-%, bezogen auf das Gesamtgewicht der Zubereitung, enthält.

9. Zubereitung oder Verwendung nach einem der vorhergehenden Ansprüche, **dadurch gekennzeichnet, dass** die kosmetische Zubereitung UV-B-Lichtschutzfilter und/oder Breitbandfilter in einer Gesamtmenge von 0,1 bis 30 Gewichts-%, bezogen auf das Gesamtgewicht der Zubereitung, enthält.

10. Zubereitung oder Verwendung nach einem der vorhergehenden Ansprüche, **dadurch gekennzeichnet, dass** die kosmetische Zubereitung in Form einer O/W-Emulsion vorliegt.
